# EUROPEAN PATENT APPLICATION

(11) **EP 2 505 658 A1**
(43) Date of publication of application: **03.10.2012**
(21) Application number: 11160717.2
(22) Date of filing: 31.03.2011
(51) Int. Cl.: C12Q 1/00

(54) **Biosensor for measuring GABA**

(71) Applicant: Universität Potsdam, 14469 Potsdam-Golm (DE)
(72) Inventor: Badalyan, Artavazd, 14467 Potsdam (DE); Leimkühler, Silke, 14471 Potsdam (DE); Stiba, Konstanze, 10247 Berlin (DE); Wollenberger, Ulla, 13187 Berlin (DE)
(74) Representative: Lange, Sven

(57) **Abstract**

The invention relates to a biosensor for measuring the concentration of GABA, wherein the sensor comprises at least two enzymes entrapped in one or two layers.

## Description

### Field of Invention

The invention relates to a biosensor for measuring a concentration of y-aminobutyric acid (GABA), wherein the sensor comprises a transaminase and an oxidoreductase. Furthermore the invention relates to the use of the biosensor for determining the in-vivo concentration of GABA and a method for the measurement of GABA.

### Background of the invention

In recent years, analytical detection in physiological fluids, e.g. blood, urine, sweat, serum, plasma etc., is of ever increasing importance in a variety of applications including clinical laboratory testing, home testing, etc. These kinds of tests have a great impact on the diagnosis and management of various disease conditions. The development of the glucose biosensor is one of the most successful testing devices to date. Although blood glucose is clearly the most important clinical parameter to be measured in the monitoring of diabetes, it is not the only parameter of clinical interest. Other measurements of medical relevance include glycosylated hemoglobin and ketone bodies. Glycosylated hemoglobin is an indicator of long-term blood glucose levels while the level of ketone bodies is an indicator of diabetic ketoacidosis (DKA) of a patient.

Bio-signal transmitters generally refer to a series of materials including all kinds of hormones, neurotransmitters, and other intercellular signal transmitters that are released from many kinds of tissues or cells in the body and transmit information to adjacent tissues or cells. Of them, the neurotransmitters may be generally classified into four classes, i.e., an amino acid-based class (e.g., acetylcholine, glycine, aspartic acid, glutamate, y-aminobutyric acid (GABA), and the like), an amine-based class (e.g., dopamine, adrenalin (epinephrine), noradrenalin, and the like), a peptide-based class (e.g., vasopressin, and the like.), and a fatty acid-based class (e.g., histamine, serotonin, and the like).

It has been known that the above compounds are released into synapses and participate in transmitting information between neurons. Such neurotransmitter release plays an important role in neurotransmission between neurons, and a very small change of the released amount affects the neurotransmission. Nevertheless, since the released amount is extremely small, it has been difficult as well as important to achieve highly sensitive detection and quantification of the bio-signal transmitter release.

Although there has been much research into the development of techniques of detecting and measuring the bio-signal transmitter release from bio-samples, none of the research has been successful in developing techniques that simultaneously satisfy high sensitivity together with a higher time and spatial resolution required for quantifying the release from a single cell level. Up to the present, most of the developed techniques have technical limits in directly detecting the bio-signal transmitter release from each position of the neural membrane. Only for a few signalling molecules the release could be directly be measured electrochemically.

Of the developed techniques, electrochemical methods, such as amperometry and fast cyclic voltametry, are useful for fast and highly sensitive detection of a bio-signal transmitter at an isolated position where the released bio-signal transmitter is readily oxidized or reduced. However, these methods are limited in the number of bio-signal transmitters to be detected. In order to solve this problem, there have been several attempts to improve the number of bio-signal transmitters to be detected by enzymatically converting the biosignal transmitters into easily oxidizable intermediates. However, the enzymatic conversion step of some neurotransmitters releases other neurotransmitter and thus is not selective enough to resolve individual neurotransmitters.

The most common, commercially-available, amperometric biosensors are based on oxidase enzymes such as glucose oxidase, lactate oxidase, and the like. These biosensors commonly use oxygen or mediators such as ferrocene, ferricyanide, etc., as electron acceptors that recycle the enzymes after substrate reaction. The use of dehydrogenase enzymes in biosensors, however, offer some advantages over the oxidase enzymes where the dehydrogenase enzyme based biosensors are unaffected by oxygen during the measurement. However, they require expensive cofactors (NAD(P)), which are difficult to regenerate in an electrochemical reaction.

The y-aminobutyric acid (GABA) is a major inhibitory neurotransmitter and is related with many diseases such as epilepsy, Huntington's disease, Parkinson's disease, diabetes, spinal cord injury, and so its accurate detection is of great interest. GABA has been detected mainly with chromatographic methods known in the art. GABA was separated and detected by liquid chromatography using derivatization with 2,4,6-trinitrobenzenesulfonic acid, anion exchange chromatography, or high performance liquid chromatography (HPLC) using o-phthaldialdehyde/2-mercaptoethanol (OPT) derivatization. See e.g. Caudill, et al. (1982) J. Chromatogr. 227:331-339; Wu, eta!. (1979) Neurochem. Res. 4:201-212; and Lindroth, et a!. (1979) Anal. Chern. 51:1667-1674. GABA localized in brain has been measured with 1 H Nuclear magnetic resonance (NMR) spectroscopy. See Rothman, et al. (1993) PNAS USA 90:5662-5666. Unfortunately, the direct and real-time detection of GABA is difficult because GABA is insensitive to electrochemical and spectrophotometrical methods. U.S. Patent Application Publication No. 20050173267 discloses a cumbersome gadget, an acoustical immunosensor, having a plurality of electrodes, piezoelectric materials, impedance analyzers, and the like for the real-time detection of GABA. Also a crude enzyme extract called GABASe, which is a mixture of a γ-aminobutyric glutamic transaminase (GABA-T) and succinic semialdehyde dehydrogenase (SSDH) is applied for sensing. In a biosensors GABAse and a NADH oxidase HPO on a tip of an oxygen sensitive electrode were applied. The resulting sensor is not very sensitive and can only measure GABA in the presence of NAD(P) cofactor (Manzei et al.: Peroxidase based amperometric biosensors for the determination of γ-aminobutyric acid, Anal. Chim Acta 328, 1996, 41). On the other hand endogeneous NADh is causing erroneous signals. Furthermore measurements are disturbed by variations of the oxygen concentration in the entire compartment near the electrode.

It was an objective of the invention to provide a device or a method which overcomes the problems known in the state of art and allows an accurate determination of a GABA concentration.

### Summary of the Invention

The present invention relates to a device especially a biosensor and a method for assaying complex cellular pathways including the y-aminobutyric acid (GABA).

Surprisingly the invention solves the underlying problem by providing a biosensor for measuring a concentration of γ-aminobutyric acid (GABA), comprising a transaminase and a oxidoreductase, entrapped in a penetrable layer.

The bio-signal transmitter γ-aminobutyric acid is chemically an amino acid, however it is not incorporated into proteins. A transaminase is preferably an enzyme that catalyzes a reaction between an amino acid and an α-keto acid. To be specific, this reaction (transamination) involves removing the amino group from the amino acid, leaving behind an α-keto acid, and transferring it to the reactant α-keto acid and converting it into an amino acid.

The oxidoreductase is preferably an enzyme that catalyzes the transfer of electrons from one molecule (the reductant, also called the hydrogen or electron donor) to another (the oxidant, also called the hydrogen or electron acceptor). This group of enzymes usually utilizes NADP or NAD as cofactors and referred to as redox enzymes. However, it was very surprising that the invention allows the sequential conversion of GABA into a dicarboxylic acid without the need of oxygen or a co-factor such as NADPH. Furthermore, due to the combination of the enzymes and the layer it is possible to perform in-vivo and in-vitro measurements of GABA, whereby the measurement is not effected by interfering substances such as glutamate and is oxygen insensitive.

The present invention is based on the discovery that a transaminase and an oxidoreductase can be used in one or more layers to measure the concentration of GABA without the need of NAD(P)H. Surprisingly, the concentration of GABA can be measured in-vivo or in-situ. The in-vivo measurement of GABA relates to introducing the biosensor into a body, wherein the body can be chosen from the group comprising an animal, human or any other dead or living body, such as an object containing a fluid. In order to obtain a sample, the biosensor must be inserted into the body to reach the appropriate compartment for sampling analytes of interest. The site of sampling could be artery, solid tissue or a cavity of the body, but is preferable brain compartment. The biosensor can also be used for the in-situ or in-vitro measurement of GABA, where a sample such as a body fluid sample is added to the biosensor or the biosensor is placed close to cultured neurocells or tissue slices in a cell or tissue cultivation chamber.

The biosensor of the present invention can also be designated as an enzyme biosensor, which can be used for assaying for the presence of an analyte in a liquid sample. The sample can be a complex biological sample such as a biological fluid and the analyte can be a naturally occurring metabolite such as GABA. The biosensors of the present invention generally can be used as multi-use sensor or a single-use strip. The single-use strip has a working electrode with a reagent matrix containing two enzymes, co-factor of one enzyme, and preferably a mediator with a redox potential above -200 mV (against Ag/AgCl) for generating a current indicative of the level of the analyte and preferably a reference/counter electrode. The reagent matrix can be in one or more layers associated with the working electrode.

The detection of GABA is preferably preformed in a sequential enzymatic reaction. The first enzyme to be in contact with the liquid containing GABA is a transaminase, preferably the GABA transaminase. The transaminase is entrapped within a layer of the biosensor and catalyzes in the presence of alpha-ketoglutarate the transamination of GABA to succinsemialdehyde. The second enzyme, which is preferably entrapped within the same layer or a second layer, catalyzes the reaction from succinsemialdehyde to a dicarboxylic acid by the oxidation of succinsemialdehyde. The second enzyme is an oxidoreductase, preferably PaoABC (Periplasmatic aldehyde oxidoreductase from E. coli). The reaction can be visualized electrochemically or optochemically. It is preferred that the reaction is visualized with a redox mediator or mediator-free.

The mediator is preferably a redox mediator, which can be entrapped within the layer. However, the mediator can also be a solution, that can be added to the layer or the enzymes. In a preferred embodiment, the layer containing the enzymes is embedded in a solution containing the redox mediator. The biosensor contains preferably one or more redox mediator(s) and one or more redox enzyme(s), which are preferably used in conjunction with one or more electrode(s). The redox mediator is especially a molecule which can shift between oxidized and reduced states and thereby facilitate electron transfer between the reactive centre of an enzyme and an electrode surface. Such mediators as ferricyanide, phenoxazines, phenothiazines, tetrathiafulvalene, ferrocene derivatives, quinones and bipyridinium salts. For a continuous or semi-continuous shuttling of electrons between the electrode and the mediator, it is essential that the electron mediator does not leach from the vicinity of the electrode.

It can be preferred to use redox polymers instead of a redox mediator. Surprisingly, the redox polymers overcome the leaching problem with additional advantages. Typically, a redox polymer consists of a system where a redox-active transition metal based pendant group is covalently bound to some sort of polymer backbone, which may or may not be electroactive. Certain redox mediators can also be polymerised or cross-linked with/without other monomers.

It can preferred to use conductive polymers as redox polymers. Conductive polymers are of repeat units with delocalized electron states. Such polymers are, e.g., polythiophenes, polyanilines or polypyrrols.

The biosensor includes an electrode body that can be preferentially of Pt, Au, metal oxide, carbon, spectroscopic graphite, pyrolytic graphite, glassy carbon and a reference electrode, separated by an electrolyte. The electrode has a needle type body, a cylindrical shape with an electro active disk. The biosensor includes preferably an elongated electrically insulating carrier having longitudinal, substantially parallel, electrically conducting tracks. The carrier can be a ceramic substrate or silicon/ silicon oxide, a plastic or paper support preferentially in a chip formate, e.g. 1 x 3 x 0.1 cm (length x width x thickness).

Each track is provided at the same end with means for electrical connection to a read-out and an electrode at the other end. One of the electrodes is the reference/counter electrode and another is the working electrode with test reagent. The sensor can be configured in the form of a supporting strip of electrically insulating carrier material such as a synthetic polymer carrying the working and reference electrodes supported on electrically conductive tracks between its ends. For example, the electrodes can take the form of two shaped areas side by side on the carrier strip. Such areas can be designed as a target area to be covered by a quantity of sample, such as whole blood, for testing the analyte. The sample areas can be circular, rectangular, diamond-shaped, semicircular, or triangular areas and can be employed to provide a target area for optimized contact by a liquid sample. It is also preferred for in-vivo measurements that the sensor has a shape of a injection needle or is placed in an injection needle, where e.g. the tip of the body contains a noble metal, metal oxide or carbon in connection with a reference electrode.

Other electrodes such as a compensating electrode can also be included. These other electrodes can be of similar formulation to the working electrode (i.e., with the associated test reagents), but lacking one or more of the working electrode's active components. A compensating electrode, for example, can provide more reliable results, in that if charge passed at the compensating electrode is subtracted from charge passed at the working electrode, then the resulting charge can be concluded to be due to the reaction of interest.

The reference electrode/counter electrode may be a classic silver/silver chloride electrode but it may also be identical to the working electrode in construction. In one embodiment, the two separate conductive tracks may both be coated with an appropriate formulation of enzyme, cofactor and mediator in a binder containing aqueous solution to yield a coating/reagent matrix. In those cases in which the coating is non-conductive, a common coating may overlay both electrodes. When a potential is applied one of the electrodes will function as a reference/counter electrode by absorbing the electrons liberated at the working electrode. The mediator at the reference/counter electrode will simply become reduced as a result of interaction with the electron flow at its electrode.

It is a preferred embodiment of the present invention that the working electrode or the reagent mixture contains also glutamate oxidase to regenerate consumed alpha-keto glutarate and eliminate its limitation of the sensor signal.

In a preferred embodiment of the present invention, a working electrode is produced by using a formulation which includes not only the enzyme, the enzyme cofactor and the mediator but also binder ingredients which cause the working electrode to give a more consistent response to concentrations of interest for the analyte being sensed. A reaction layer on the surface of the working electrode is provided when the sample is applied. As the mediator is reduced by reaction with the enzyme, cofactor and analyte, it is retained in close proximity to the electrode surface so that it can be readily reoxidized. The maintenance of this thin reaction layer also allows the overall analytical reaction to occur in a small volume of the overall sample.

It is preferred that the generated signal is a function of the analyte concentration. The signal may be the current observed at a fixed time after the test is initiated or it may be the current integrated over some period occurring some fixed time after the test is initiated (in essence the charge transferred over some such period). The test is conducted by covering the working electrode and a reference/counter electrode with sample and a potential applied between them. The current which then flows is either observed over some time period or measured at a set point in time after application of the potential between the electrodes. The potential may be imposed as soon as the sample covers the electrodes or it may be imposed after a short delay. The fixed time until the current or current integration is taken should be long enough to ensure that the major variable affecting the observed current is the analyte concentration.

In a further embodiment, the mediator has a concentration in a layer of about 2.0% (w/w) or less and, preferably, in the range of about 0.2% to about 1.0% (w/w). The mediator can also be bound chemically to the electrode surface or is part of the matrix polymer, preferably ferrocene, quinone or Os-complexes bound to flexible linkers such as ethylene glycols. It is also preferred that mediators of low solubility, e.g. tetrathiafulvalene or dimethyl ferrocene are enriched on the electrode surface. It is also preferred that the redox polymer is by weight about 50 % of the total amount of material bound to the surface, preferable 50 µg of Poly(vinylpyridine)-[osmium-(N,N'-methylated-2,2'-biimidazole)3] and 25 µg oxidoreductase and 25 µg transaminase.

In still another embodiment of the present invention, the mediator is one of meldola's blue, 1-methoxy phenazine methosulfate, 1,2-benzoquinone, and 2,6-dichloroindophenol. It can be preferred that the mediator is immobilized within a layer.

The enzymes are preferably entrapped within one or two layer, wherein the enzymes can be incorporated into one layer or two separated layers. The layer is preferably a gel, such as a hydrogel. A hydrogel is defined as a gel which contains water but is not soluble in water. The starting polymers for the gel formation comprise gelatin, carrageenan, chitosan, alginic acid, poly (2-hydroxyethyl methacrylate), carboxylated methylstarch, hydrolyzate of acrylonitrile-grafted starch, polyacrylamide, poly(acrylic acid) salt, hydrolyzate of vinyl acetatemethyl acrylate copolymer, polyoxyethylene, polyurethane, poly(vinyl pyrrolidone), polystyrene sulfonate, poly(vinyl alcohol) and so on. It is a well-known that the viscosity of highly concentrated aqueous solutions of poly(vinyl alcohol) (hereinafter referred to as PVA) increase with time and the solutions finally set to a gel when allowed to stand at room temperature. The resultant gel is sticky and low in mechanical strength.

A variety of methods have been reported to enhance the mechanical strength of the hydrated PVA gel. Among them are chemical crosslinking of PVA with aldehydes such as formaldehyde and glutaraldehyde, PVA crosslinking by irradiation with radiations such as gamma rays, electron beams, and ultraviolet light, PVA crosslinking through coordinate bonding with metal ions such as Ti, Cu, and Co, and PVA crosslinking with the use of boric acid, borax, and Congo Red. In a preferred embodiment the gel forming the layer is made of polyvinylalcohol.

The invention furthermore provides a method for measuring an γ-aminobutyric acid concentration comprising A method for measuring an γ-aminobutyric acid concentration comprising contacting a liquid with the biosensor, in which a transaminase, preferably an γ-aminobutyric acid transaminase and a reductase, preferably an aldehyde oxidoreductase are incorporated, wherein a first reaction product is generated by a desamination of y-aminobutyric acid by the transaminase, and wherein a second product is generated by an oxidation of the first product by the aldehyde oxidoreductase.

### Brief Description of the Figures

The accompanying drawings are included to provide a further understanding of embodiments. Other embodiments and many of the intended advantages will be readily appreciated as they become better understood by reference to the following detailed description. The elements of the drawings do not necessarily scale to each other. Like reference numbers designate corresponding similar parts. The invention is illustrated in the following figures, in which
- Fig. 1: is a schematic drawing of biosensor
- Fig. 2: is a enlarged view of the biosensor
- Fig. 3: is a view of the reaction sequence occurring in the biosensor
- Fig. 4: is a diagram showing the output of the biosensor

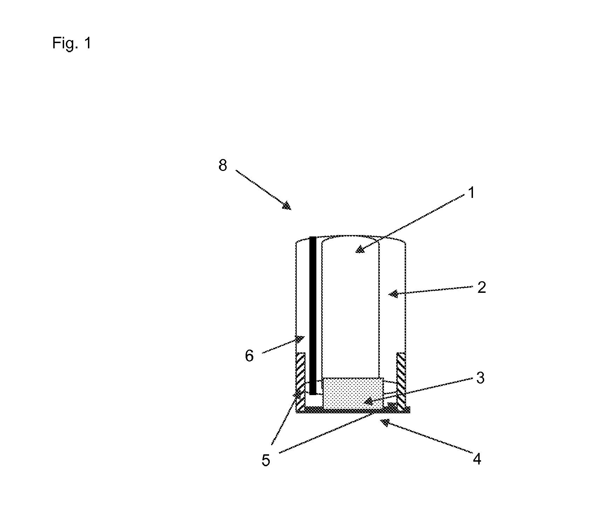

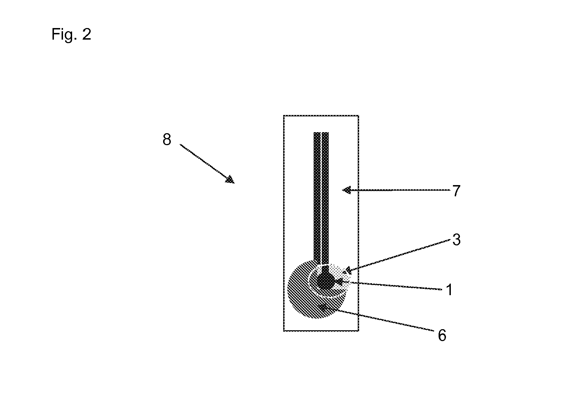

Fig. 1 and Fig. 2 show schematic drawing of a preferred biosensor. The biosensor 8 contains preferably an electrode body 1, which is surrounded by an electrode insulation 2. The layer 3 comprising the enzymes is mounted onto the electrode. However, the layer can be operatively connected with the electrode in various ways. At least two enzymes are entrapped within the layer 3; a transaminase and an oxidoreductase. The oxidoreductase is preferably an Idehyde oxidoreductase, wherein the transaminase is preferably a GABA transaminase. Furthermore, a mediator can also be entrapped within the layer 3. The mediator is preferably a molecule, that is able to migrate through the layer 3, but can also be immobilized in the layer 3 or a solution which is contacted by the layer 3. The biosensor 8 further comprises a protection layer 4 and an O-ring 5 for protection. The electron transfer from the mediator to an electrode 1 and the subsequent change of the potential of the electrode is measured by a reference electrode 6. The biosensor 8 also contains a carrier substrate 7.

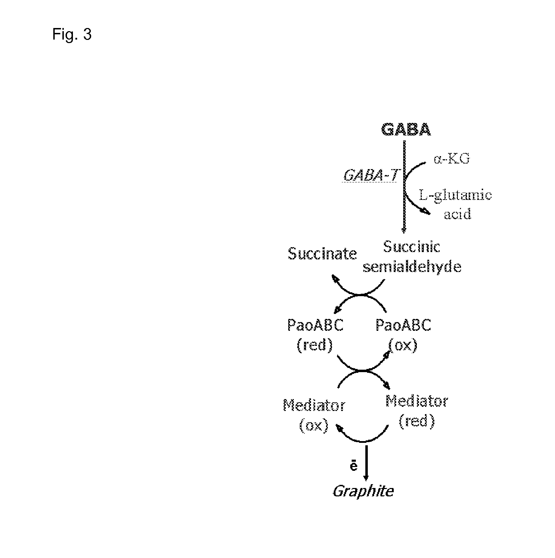

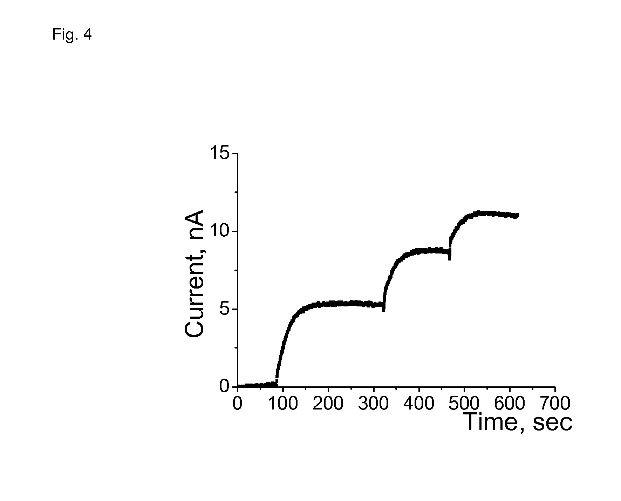

Fig. 3 shows the reaction sequence preferably occurring in the biosensor, wherein Fig. 4 shows the response of the biosensor. Response of a spectrographic graphite electrode onto which a PVA-gel membrane prepared with 1 U GABA-T and 2 U/cm2 in 5 % PVA-gel and stabilized by UV-crosslinking has been fixed by a cellulose acetate membrane. A potential of +50 mV against Ag/AgCl 1 M KCI is applied. The electrode is in contact with 5 mM phenazine methosulfate, 2 mM alpha-keto glutarate in 0.1 M TRIS (pH 7), 0.1 KCI. The Fig. 4 shows chronoamperometric response of the mediator oxidation recorded upon injection of 200µM GABA. The reaction sequence is preferably: GABA + alpha-ketoglutarate → succinic semialdehyde + glutamate; Succinic semialdehyde + oxidized mediator → Succinic dicarbonic acid - reduced mediator

GABA is reacting with an enzyme, for example the GABA-transaminase, which catalyzes the reaction from GABA to succinic semialdehyde in the presence of alpha-ketoglutaric acid. The succinic semialdehyde is then oxidized by an enzyme, for example the aldehyde oxidoreductase to succinate, wherein the enzyme is regenerated by a redox mediator. In the final step, the mediator transfers electrons to an electrode, which can be measured by an amperometric method.

### Reference list

- 1: Electrode body
- 2: Electrode insulation
- 3: Layer
- 4: Protection Layer
- 5: O-ring
- 6: Reference electrode
- 7: Carrier substrate
- 8: Biosensor

## Claims

1. Biosensor for measuring a concentration of γ-aminobutyric acid, comprising at least a transaminase and a oxidoreductase, on an electrode entrapped in a penetrable layer.

2. Biosensor according to claim 1, wherein the transaminase is an γ-aminobutyric acid transaminase.

3. Biosensor according to claim 1 or 2, wherein the NADH independent oxidoreductase is an aldehyde oxidoreductase.

4. Biosensor according to at least one of the preceding claims, wherein the biosensor comprises at least one layer.

5. Biosensor according to at least one of the preceding claims, wherein the layer is a gel.

6. Biosensor according to at least one of the preceding claims, wherein the layer or the contacting solution comprises at least one redox mediator.

7. Use of the biosensor according to claim 1 to 6 for an in-vivo or ex-vivo measuring of an γ-aminobutyric acid concentration.

8. A method for measuring an γ-aminobutyric acid concentration comprising contacting a liquid with the biosensor, in which a transaminase, preferably an γ-aminobutyric acid transaminase and a reductase, preferably an aldehyde oxidoreductase are incorporated, wherein a first reaction product is generated by a desamination of γ-aminobutyric acid by the transaminase, and wherein a second product is generated by an oxidation of the first product by the aldehyde oxidoreductase.

9. Method according to claim 8, wherein the aldehyde oxidorectase is regenerated by an electrode or a redox mediator.

10. Method according to claim 8 or 9, wherein the regeneration is visualized electrochemically or optochemically.
